# EUROPÄISCHE PATENTSCHRIFT

(11) **EP 1 140 754 B1**
(45) Veröffentlichungstag und Bekanntmachung des Hinweises auf die Patenterteilung: **25.01.2006**
(21) Anmeldenummer: 00902563.6
(22) Anmeldetag: 05.01.2000
(51) Int. Cl.: C07C 39/16, B01J 2/04

(54) **VERFAHREN UND VORRICHTUNG ZUR HERSTELLUNG VON BISPHENOL-A-PRILLS UND DANACH HERGESTELLTE BISPHENOL-A-PRILLS**
METHOD AND DEVICE FOR PRODUCING BISPHENOL A PRILLS AND BISPHENOL A PRILLS PRODUCED ACCORDING TO THIS METHOD
PROCEDE ET DISPOSITIF DE PRODUCTION D'UTRICULES DE BISPHENOL A ET UTRICULES DE BISPHENOL A AINSI PRODUITS

(30) Priorität: 07.01.1999 DE 19900221
(43) Veröffentlichungstag der Anmeldung: 10.10.2001
(73) Patentinhaber: Bayer MaterialScience AG, 51368 Leverkusen (DE)
(72) Erfinder: LANZE, Rolf, D-47804 Krefeld (DE); EITEL, Alfred, D-41539 Dormagen (DE); NEUMANN, Rainer, D-47803 Krefeld (DE); KÜHLING, Steffen, B-9100 Sint Niklaas (BE); HEYDENREICH, Frieder, D-40593 Düsseldorf (DE); VAN OSSELAER, Tony, D-47800 Krefeld (DE); BELLINGHAUSEN, Rainer, D-29629 Bomlitz (DE); HEROLD, Heiko, D-41470 Neuss (DE)
(86) Internationale Anmeldenummer: PCT/EP2000/000041
(87) Internationale Veröffentlichungsnummer: WO 2000/040533

(56) Entgegenhaltungen:
- EP-A- 0 278 246
- PATENT ABSTRACTS OF JAPAN vol. 018, no. 385 (C-1227), 20. Juli 1994 (1994-07-20) & JP 06 107581 A (NIPPON STEEL CHEM CO LTD;OTHERS: 01), 19. April 1994 (1994-04-19) in der Anmeldung erwähnt

## Beschreibung

Die Erfindung betrifft ein Verfahren zur Herstellung von Bisphenol-A-Prills, wobei schmelzflüssiges Bisphenol-A am Kopf eines Prillturmes über eine Düsenplatte mit einer Vielzahl von Düsen in den Prillturm eingetragen wird, in den im Gegenstrom ein im Kreislauf geführtes Kühlgas eingeleitet wird und wobei das auf etwa Raumtemperatur abgekühlte Bisphenol-A-Prill am Fuß des Prillturmes gesammelt und abgezogen wird und darüber hinaus eine Vorrichtung zur Durchführung eines solchen Verfahrens sowie ein nach diesem Verfahren hergestellte Bisphenol-A-Prills.

Bisphenole sind durch Umsetzen von Phenol mit Ketonen gewonnene chemische Verbindungen mit zwei Phenolgruppen, wobei Bisphenol-A (2,2- Bis(4-hydroxyphenyl)propan)) durch die Umsetzung von Phenol und Aceton entsteht. Bisphenol-A wird zu Epoxidharzen, Polycarbonaten und Polysulfonen weiterverarbeitet. Zur Erleichterung von Abfüllung, Transport und Lagerung werden aus einer Bisphenol-Schmelze durch Abkühlung Granulat, Schuppen oder Prills hergestellt, wobei Prills aufgrund des geringeren Staubanteils und der besseren Fließeigenschaften Vorteile gegenüber Granulat bzw. Schuppen aufweisen.

Aus der JP-6-107 581 ist ein Verfahren zur Herstellung von Bisphenol-A-Prills bekannt, bei dem schmelzflüssiges Bisphenol-A im Kopfbereich eines Prillturmes eingetragen wird und im Gegenstrom am Fuße des Prillturmes Kühlgas eingebracht wird, das den herunterfallenden Schmelzetröpfchen die Schmelzwärme entzieht. Am Fuße des Prillturmes werden die verfestigten Prills abgezogen. Ferner ist aus JP 6 107 580 ein Verfahren zur Herstellung von Bisphenol-A-Prills bekannt, bei dem das radial in den Kühlgasverteiler eintretende Gas mit Hilfe eines Trennblechs in dem Verteilergehäuse tangential um den Prillturm geleitet wird. Eine Teilmenge des Gases wird in vertikaler Richtung zum Konus am Fuß des Prillturms geleitet. Das Kühlgas wird im Konusbereich um 180° umgelenkt und gleichmäßig über den gesamten Querschnitt verteilt aufwärts geführt.

Ziel des bekannten Verfahrens war es, die Härte der hergestellten Bisphenol-A-Prills zu erhöhen, um den Staubanteil zu verringern. Je nach Art der Weiterverarbeitung werden jedoch, neben der Staubarmut, noch weitere Anforderungen an die Prills gestellt.

Der vorliegenden Erfindung liegt daher die Aufgabe zugrunde, ein Verfahren bzw. eine Vorrichtung zur Herstellung von Bisphenol-A-Prills und entsprechend hergestellte Prills zu schaffen, bei denen die Produktreinheit im Vordergrund steht. Zum Erhalt von möglichst reinen Bisphenol-A-Prills soll einerseits deren Monophenolgehalte und Staubrestgehalt reduziert und andererseits ein besonders klares und auch farbstabiles Produkt geschaffen werden.

Diese Aufgabe wird hinsichtlich des Verfahrens dadurch gelöst, dass der Eintrag der Bisphenol-A-Schmelze und die Einleitung des Kühlgases gleichmäßig über den Querschnitt des Prillturmes verteilt erfolgt.

In einer besonderen Ausführungsform der vorliegenden Erfindung hat die Bisphenol-A-Schmelze beim Eintrag eine Temperatur von 160°C bis 170°C.

Vorrichtungsmäßig erfolgt die Lösung dadurch, dass zur gleichmäßigen Kühlgasaufgabe eine horizontal in den Prillturm geführte und sich im Querschnitt verjüngende Ringraumleitung vorgesehen ist, dass im radial angeflanschten Kühlgaskanaleintritt ein Trennblech zum Teilen des Kühlgasstromes in zwei Teilströme vorgesehen ist und dass eine Vielzahl gleichmäßig über den Umfang des Prillturmes angeordneter wabenartiger Gleichrichterelemente vorgesehen sind. Auf diese Weise ist es erfindungsgemäß möglich, das Kühlgas drallfrei und gerichtet auf den Querschnitt des Prillturmes zu verteilen.

Mit dem erfindungsgemäßen Verfahren bzw. der erfindungsgemäßen Vorrichtung lassen sich Prills herstellen, deren Staubanteil unter 2 Massen-% liegt, die eine Eigenfarbe von kleiner 10 Hazen besitzen und die eine BET-Oberfläche von >0,15 m²/g aufweisen. Die Partikelgröße dp liegt dabei zwischen 0,5 und 3 mm, bevorzugt zwischen 0,8 und 2 mm.

Nachfolgend wird das erfindungsgemäße Verfahren näher beschrieben. Wesentlich für das Verfahren der phenol- und staubarmen Herstellung von Bisphenol-A-Prills ist eine möglichst exakte Temperatureinstellung der Bisphenol-A-Schmelze für die Verdüsung in einem weiten Arbeitsbereich. Diese Temperierung wird bevorzugt mittels eines Rohrbündelaustauscher ermöglicht. Die Bisphenol-A-Schmelze fällt im Verfahren vorzugsweise bei 185°C bis 250°C an. Im vorgenannten Rohrbündelaustauscher wird die Bisphenol-A-Schmelze bis nahe an die Kristallisationstemperatur von 156°C abgekühlt. Bevorzugt beträgt die Temperatur der in den Prillturm eingetragenen Bisphenol-A-Schmelze 165°C.

Zweckmäßigerweise wird zur Temperierung gemäß einer weiteren Lehre der Erfindung ein 3-Kammer-Rohrbündelaustauscher eingesetzt, wobei die Schmelze zur Abkühlung durch die Rohre des Rohrbündelaustauscher geleitet wird. Im Außenraum um die Rohre wird Druckwasser eingespeist und verdampft, um der Bisphenol-A-Schmelze Wärme zu entziehen. Die Einstellung der Temperatur der Schmelze erfolgt sowohl durch den Verdampfungsdruck des Wassers als auch über die Anzahl der mit Druckwasser abgetauchten Rohre. Da die Kühlerseite des Wärmeaustauschers unterhalb der Kristallisationstemperatur des Bisphenol-A betrieben wird, ist der Wärmeaustauscher mit einem zusätzlichen Heizmantel ausgestattet. In diesen wird während der kritischen Phasen, also beispielsweise beim Anfahren oder im Niederlastbetrieb durch Einspeisung eines Wärmeträgers das produktseitige Einfrieren zuverlässig verhindert.

In weiterer Ausgestaltung der Erfindung erfolgt unmittelbar vor dem Eintrag der Bisphenol-A-Schmelze in den Prillturm eine Reinigung der Schmelze in einem Schmelzefilter. Für diese Filtration werden vorzugsweise Metallgewebesiebe mit einer Maschenweite von <80 µm eingesetzt.

In weiterer Ausgestaltung der Erfindung wird die Düsenplatte für den Schmelzeeintrag unter einem geringen Überdruck im Bereich von 0,05 bis 1 bar, vorzugsweise zwischen 0,15 und 0,3 bar betrieben. Der Differenzdruck wird im wesentlichen durch die Geometrie der Bohrungen, der Anzahl der Bohrungen pro Düsenplatte, der Temperatur der Schmelze und der Einspeisemenge (Volumenstrom) bestimmt. Zur gleichmäßigen Verteilung der Bisphenol-A-Schmelze ist die Düsenplatte bevorzugt sphärisch gewölbt ausgebildet und zentral auf dem Prillturmkopf angeordnet. Darüber hinaus sind in weiterer Ausgestaltung der Erfindung im oberen Teil des Prillturmes mehrere, gleichmäßig über den Querschnitt des Prillturmes verteilte Entnahmerohre angeordnet, über die das Kühlgas abgezogen wird. Zweckmäßigerweise sind diese Entnahmerohre begleitbeheizt ausgeführt. Auf diese Weise lassen sich Produktablagerungen zuverlässig verhindern.

Aufgrund der Tatsache, dass das Kühlgas im Kreislauf gerührt wird, muß eine Reinigung des Kühlgases erfolgen, die bevorzugt als Schlauchfilter ausgeführt ist.

Eine weitere Lehre der Erfindung sieht vor, dass das im Kreislauf geführte Kühlgas kontinuierlich gekühlt und von Monophenolen befreit wird, wobei die Kühlung vorzugsweise in einem Waschturm mit Sekundärkühlkreislauf erfolgt. Hierdurch wird die Baugröße des Kreislaufkühlers deutlich verringert und auch der kontinuierliche Betrieb ist erst durch diese Ausgestaltung möglich, da bei zur Kühlung eingesetzten Rohrbündelaustauschem diese stets nur als Umschaltaustauscher eingesetzt werden können, um das Ausfrieren des Phenols aus dem Kreislaufstickstoff zu ermöglichen.

Bevorzugt erfolgt die Kühlung des im Kreislauf geführten Kühlgases mit demineralisiertem Wasser ohne gelösten Sauerstoff, um eine Schaumbildung zuverlässig zu vermeiden.

Die erfindungsgemäße Vorrichtung wird nachfolgend anhand einer ein bevorzugtes Ausführungsbeispiel darstellenden Zeichnung näher erläutert. In der Zeichnung zeigen
- Fig. 1: eine schematische Darstellung des erfindungsgemäßen Verfahrens zur Herstellung von Bisphenol-A-Prills,
- Fig. 2: den Fußbereich des Prillturmes im Querschnitt,
- Fig. 3: den Fußbereich des Prillturmes im Horizontalschnitt in schematischer Darstellung,
- Fig. 4: einen Querschnitt durch die zur Schmelzeeindüsung verwendete Düsenplatte,
- Fig. 5: die schematische Anordnung von Kühlgasentnahmerohren und Schmelzeeintrag am Prillturmkopf und
- Fig. 6: einen Schmelzekühler zur Temperierung der einzutragenden Bisphenol-A-Prills schematisch im Querschnitt.

In Fig. I ist eine Übersicht der erfindungsgemäßen Vorrichtung zur Herstellung von Bisphenol-A-Prills mit Kühlgas-Kreislaufführung dargestellt. In einen Prillturm 1 wird die in einem nicht dargestellten Schmelzekühler temperierte BPA-Schmelze über eine nur schematisch dargestellte Düsenplatte 2 verdüst eingetragen. Um den gesamten Prillturmquerschnitt für den intensiven Wärme- und Stoffaustausch zwischen Bisphenol und Kühlgas auszunutzen, ist die Düsenplatte 2 zentral auf dem Kopf des Prillturmes 1 angeordnet. Kühlgas, bevorzugt Stickstoff (N₂) wird am Fuße des Prillturmes 1 über einen Kühlgasverteiler 3 im Gegenstrom eingeleitet. Dadurch werden die eingetragenen BPA-Schmelztröpfchen abgekühlt und zu Bisphenol-A-Prills verfestigt. In einem Konus am Fuß des Prillturmes 1 werden die Prills gesammelt, über ein Sieb 4 von Grobteilchen befreit und über eine beispielsweise pneumatische Förderanlage 5 in Lagersilos transportiert. Das Überkorn wird dabei in einem Behälter 6 gesammelt. Das erwärmte Kühlgas wird am Kopf des Prillturmes über mehrere Entnahmerohre 7 entnommen und in einem Feststoffilter 8 von mitgerissenem Feinststaub befreit. Der Feinststaub wird wie das Überkorn zur Weiterverwendung in einem Staubbehälter 9 gesammelt.

Anschließend wird das Kühlgas in einem Waschturm 10 gekühlt und durch intensiven Kontakt mit der Waschflüssigkeit von den abgestrippten, leicht flüchtigen Monophenolen befreit, bevor es als Kühlgas wieder dem Prillturm 1 zugeführt wird. Der Kreislaufbetrieb wird mit Hilfe eines Ventilators 11 aufrechterhalten.

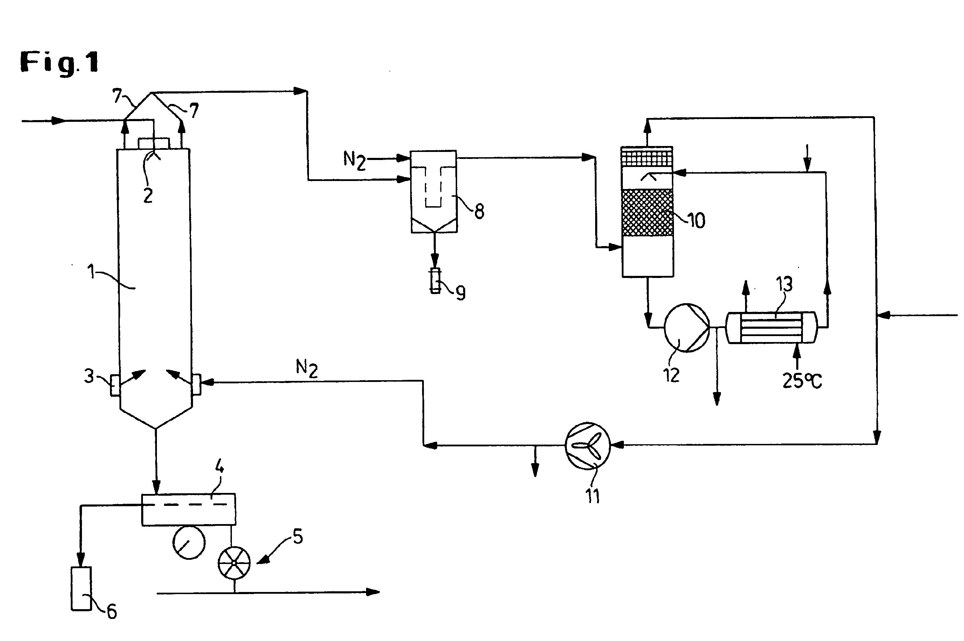

Wie aus Fig. 1 ersichtlich, wird die Kühlflüssigkeit des Waschturmes 10 bevorzugt ebenfalls im Kreislauf betrieben. Die erwärmte Waschflüssigkeit wird mittels einer Pumpe 12 einem Wärmetauscher 13 zugeführt, dort abgekühlt und anschließend zum Kopf des Waschturmes 10 geleitet. Um eine Anreicherung an ausgewaschenen Phenolen zu vermeiden, wird aus dem Kühlkreislauf ständig eine Teilmenge ausgeschleust und durch frische Waschflüssigkeit ersetzt, wie mit den beiden Pfeilen dem Waschflüssigkeitskreislauf angedeutet ist.

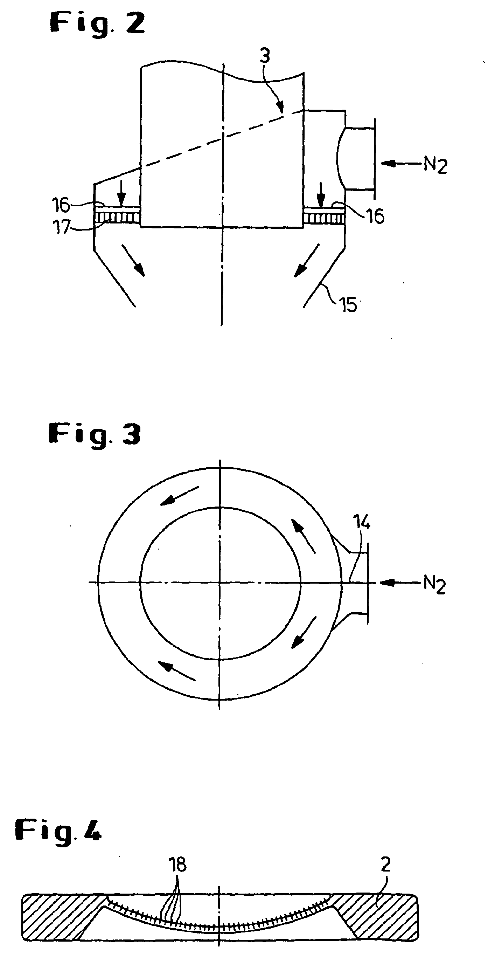

Zur optimalen Abkühlung und Abstrippung der Bisphenol-Schmelze ist ein gleichmäßiger intensiver Kontakt zwischen Kühlgas und Schmelzetropfen erforderlich. Besonders wichtig ist daher das schnelle Erreichen einer ausgebildeten, aufwärts gerichteten, drallfreien Strömung des Kühlgases im Prillturm 1. Dies wird durch den in den Fig. 2 und 3 gezeigten erfindungsgemäßen Kühlgasverteiler 3 erreicht.

Der Kühlgasstrom tritt dabei radial in den Kühlgasverteiler 3 ein und wird durch ein Trennblech 14 in zwei gleich große Komponenten aufgeteilt. Beide Ströme werden in einem Verteilergehäuse tangential um den Prillturm 1 geleitet. In Umfangsrichtung des Prillturmes 1 wird dabei stetig eine Teilmenge des Kühlgases in vertikaler Richtung zum Konus 15 am Fuß des Prillturmes 1 abgeführt. Lochbleche 16 sorgen für die Gleichverteilung der abströmenden Kühlgasmenge über den Umfang des Prillturmes 1. Ferner wird die Gleichverteilung durch eine stetige, in Fig. 2 deutlich sichtbare Querschnittsverjüngung des Verteilerkanals um den Prillturm 1 unterstützt.

Unterhalb der Lochbleche 16 sind erfindungsgemäß Gleichrichterelemente 17 angeordnet, deren bevorzugtes Längen/Durchmesser-Verhältnis größer als 5 ist. Durch den Einsatz dieser zweckmäßigerweise aus wabenförmigen zu Gleichrichterpackungen zusammengestellten Gleichrichterelemente 17 wird eine nach unten gerichtete gleichmäßige Strömung zum Konus 15 bewirkt. Im Konusbereich wird die Kühlgasmenge um 180° umgelenkt und strömt nach einer kurzen Einlaufstrecke gleichmäßig über den gesamten Querschnitt verteilt aufwärts durch den Prillturm 1.

Eine bevorzugt sphärisch ausgebildete Düsenplatte 2 ist in Fig. 4 dargestellt. Eine Vielzahl von Bohrungen 18 ist dabei gleichmäßig auf der Oberfläche der Düsenplatte 2 verteilt. Wie bereits ausgeführt, wird die Düsenplatte 2 mit einem geringen Differenzdruck betrieben, um die geforderte Restphenol- und Staubspezifikationen gewährleisten zu können. Da sich der Differenzdruck im wesentlichen durch die Geometrie der Bohrungen 18, der Anzahl der Bohrungen 18 pro Düsenplatte 2, der Temperatur der BPA-Schmelze und der Einspeisemenge bestimmt, sind zweckmäßigerweise für unterschiedliche Lastanpassungen auswechselbare Düsenplatten 2 vorhanden, um für jeden Lastbereich eine optimale Verteilung der Schmelzetröpfchen über den Querschnitt des Prillturmes 1 zu erreichen.

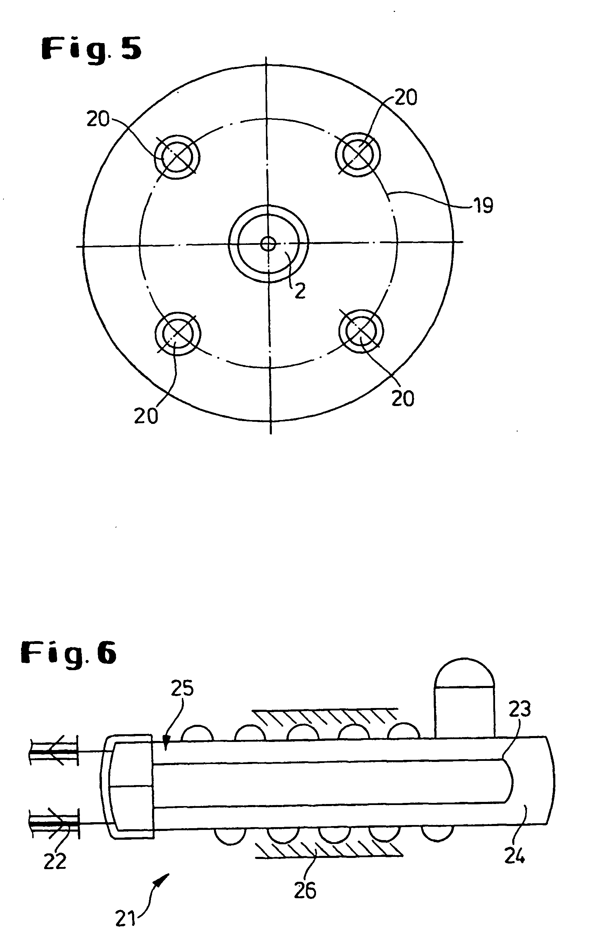

Auch der obere Bereich des Prillturmes 1 muß möglichst frei von Querströmungen sein, um den gleichmäßigen Kontakt von Kühlgas und Schmelzetropfen über der gesamten Prillturmhöhe zu gewährleisten. Dies setzt voraus, dass die gerichtete Aufwärtsströmung insbesondere auch im Kopfbereich, also im Ort der Schmelzeeindüsung, wenig gestört wird. Die Gasabfuhr am Prillturmkopf erfolgt deshalb durch mehrere, vorzugsweise drei bis vier, auf einem Lochkreis 19 symmetrisch angeordnete Entnahmerohre 20, wie schematisch in Fig. 5 gezeigt. Die Entnahmerohre 20 werden strömungsgünstig in einem nicht dargestellten Sammelrohr mit äquivalentem Querschnitt zusammengeführt. Auf diese Weise ermöglicht die zuvor beschriebene Anordnung der Entnahmerohre 20 die zentrische Eindüsung der Bisphenol-A-Schmelze mittels der Düsenplatte 2. Zweckmäßigerweise sind die Entnahmerohre 20 begleitbeheizt ausgeführt.

Schließlich ist in Fig. 6 schematisch ein für die Temperierung der BPA-Schmelze eingesetzter Schmelzekühler 21 dargestellt. Der Schmelzekühler 21 ist dabei als 3-Kammer-Rohbündelaustauscher ausgeführt. Die im Verfahren vorzugsweise bei Temperaturen zwischen 185°C und 250°C anfallende BPA-Schmelze wird über einen Eintrag 22 durch Rohre 23 des Schmelzekühlers geleitet. In dem Außenraum 24 um die Rohre 23 wird Druckwasser 25 eingespeist und verdampft. Hierdurch wird der BPA-Schmelze Wärme entzogen. Da die Kühlerseite des Wärmeaustauscher unterhalb der Kristallisationstemperatur von BPA (156°C) betrieben wird, ist der Schmelzekühler 21 mit einem zusätzlichen Heizmantel 26 ausgestattet. Durch Einspeisung eines Wärmeträgers 27 (beispielsweise Dampf) in diesen Heizmantel 26 wird das produktseitige Einfrieren während der Anfahrphase bzw. bei Niederlastbetrieb des Prillverfahrens zuverlässig ausgeschlossen.

### Beispiel

Bisphenol-A-Schmelze mit einer GC-Reinheit von 99,69 Massen-% wird kontinuierlich am Kopf des Prillturms eingetragen und gemäß dem erfindungsgemäßen Verfahren zu Bisphenol-A-Prills konfektioniert. Die so erhaltenen Bisphenol-A-Prills zeichnen sich durch einen niedrigeren Gesamtphenolgehalt (Phenol-, Isopropenylphenol- und t -Butylphenol-Gehalt) aus, wobei durch den Prillvorgang im Vergleich zur eingetragenen Bisphenol-A-Schmelze eine Reduzierung des Phenolgehalts um 40 ppm auf 10 ppm, des Isopropenylphenolgehalts um 20 ppm auf 10 ppm und des t-Butylphenolgehalts um 60 ppm auf 20 ppm erbracht werden konnte. Die Prills weisen eine BET-Oberfläche von 0,17 m²/g und eine Eigenfarbe von 5 Hazen (DIN 59 409) auf.

Die hohe Qualität der erfindungsgemäßen Bisphenol-A-Prills führt auch zu einer ausgezeichneten Lagerstabilität. Diese gute Lagerstabilität zeigt sich dadurch, dass Bisphenol-A-Prills mit einer Eigenfarbe von 5 Hz, die über einen Monat bei Tageslicht und Raumtemperatur gelagert worden sind, immer noch eine Eigenfarbe von 5 Hz besitzen. Zum Vergleich dazu zeigt eine Bisphenol-A-Schmelze mit höheren Phenolanteilen (Phenolgehalt 50 ppm, Isopropenylphenolgehalt 30 ppm und t-Butylphenolgehalt 80 ppm) nach der gleichen Behandlung hingegen eine Hz-Farbzahl von 15 Hz.

Die Vorteile von staubarmen, lagerstabilen, farbhellen Bisphenol-A-Prills mit großer BET-Oberfläche zeigen sich insbesondere bei der Weiterverarbeitung des Bisphenol-A zu Polycarbonat. Zur Herstellung von 1,022 t einer 15-%igen wäßrigen Natriumbisphenol-A-Lösung werden 154,5 kg Bisphenol-A-Prills in 867,5 kg 6,5 % NaOH unter Rühren und inerten Bedingungen (der gesamte Prozeß ist mit Stickstoff inertisiert) innerhalb von 14 min gelöst. Die Hz-Farbzahl dieser Lösung beträgt anschließend 0,9 Hz. Eine solche NaBPA-Lösung wird dann umgehend zur Herstellung von Polycarbonat nach dem (für sich bekannten) Phasengrenzflächenverfahren eingesetzt. Der YI (Yellowness-Index) des entstandenen Polycarbonats mit einer relativen Lösungsviskosität von 1,200 beträgt 1,48. Die gute Qualität eines solchen Polycarbonats führt zu einer besonders hochwertigen Qualität der daraus hergestellten Formteile.

## Patentansprüche

1. Verfahren zur Herstellung von Bisphenol-A-Prills, wobei schmelzflüssiges Bisphenol-A am Kopf eines Prillturmes über eine Düsenplatte mit einer Vielzahl von Düsen in den Prillturm eingetragen wird, in den im Gegenstrom ein im Kreislauf geführtes Kühlgas eingeleitet wird und wobei das auf etwa Raumtemperatur abgekühlte Bisphenol-A-Prill am Fuß des Prillturmes gesammelt und abgezogen wird, **dadurch gekennzeichnet, dass** der Eintrag der Bisphenol-A-Schmelze und die Einleitung des Kühlgases gleichmäßig über den Querschnitt des Prillturmes verteilt erfolgt, wobei zur gleichmäßigen Kühlgasaufgabe eine horizontal in den Prillturm (1) geführte und sich im Querschnitt verjüngende Ringraumleitung vorgesehen ist, im radial angeflanschten Kühlgaskanaleintritt ein Trennblech (14) zum Teilen des Kühlgasstromes in zwei Teilströme vorgesehen ist und eine Vielzahl gleichmäßig über den Umfang des Prillturmes (1) angeordneter wabenartiger Gleichrichterelemente vorgesehen (17) sind.

2. Verfahren nach Anspruch 1, **dadurch gekennzeichnet, dass** die Temperatur der Bisphenol-A-Schmelze beim Eintrag 160°C bis 170°C beträgt.

3. Verfahren nach Anspruch 2, **dadurch gekennzeichnet, dass** die Temperatur der Bisphenol-A-Schmelze beim Eintrag 165°C beträgt.

4. Verfahren nach einem der Ansprüche 1 bis 3, **dadurch gekennzeichnet, dass** unmittelbar vor dem Eintrag der Bisphenol-A-Schmelze in den Prillturm eine Reinigung der Schmelze in einem Schmelzefilter erfolgt.

5. Verfahren nach einem der Ansprüche 1 bis 4, **dadurch gekennzeichnet, dass** die Düsenplatte unter einem geringen Überdruck im Bereich von 0,05 bis 1 bar betrieben wird.

6. Verfahren nach Anspruch 5, **dadurch gekennzeichnet, dass** der Überdruck 0,15 bis 0,3 bar beträgt.

7. Verfahren nach einem der Ansprüche 1 bis 6, **dadurch gekennzeichnet, dass** das Kühlgas über die gesamte Höhe des Prillturmes in einem im wesentlichen gerichteten und drallfreien Strömungsverlauf geführt wird.

8. Verfahren nach einem der Ansprüche 1 bis 7, **dadurch gekennzeichnet, dass** das Kühlgas im oberen Teil des Prillturmes durch mehrere, gleichmäßig über den Querschnitt des Prillturmes verteilte Entnahmerohre abgezogen wird.

9. Verfahren nach Anspruch 8, **dadurch gekennzeichnet, dass** die Entnahmerohre begleitbeheizt sind.

10. Verfahren nach einem der Ansprüche 1 bis 9, **dadurch gekennzeichnet, dass** das im Kreislauf geführte Kühlgas mechanisch gefiltert wird.

11. Verfahren nach einem der Ansprüche 1 bis 10, **dadurch gekennzeichnet, dass** das im Kreislauf geführte Kühlgas kontinuierlich gekühlt wird.

12. Verfahren nach Anspruch 11, **dadurch gekennzeichnet, dass** die Kühlung des Kühlgases in einem Waschturm mit Sekundärkühlkreislauf erfolgt.

13. Verfahren nach Anspruch 12, **dadurch gekennzeichnet, dass** die Kühlung des im Kreislauf geführten Kühlgases mit demineralisiertem Wasser ohne gelösten Sauerstoff erfolgt.

14. Vorrichtung zur Herstellung von Bisphenol-A-Prills unter Durchführung des Verfahrens nach einem der Ansprüche 1 bis 13, mit einem Prillturm, einer Bisphenol-A-Schmelzeaufgabe, einem Kühlgaskreislauf mit Gebläse und Kühler, einem Bisphenol-A-Prill-Abzug, **dadurch gekennzeichnet, dass** zur gleichmäßigen Kühlgasaufgabe eine horizontal in den Prillturm (1) geführte und sich im Querschnitt verjüngende Ringraumleitung vorgesehen ist, dass im radial angeflanschten Kühlgaskanaleintritt ein Trennblech (14) zum Teilen des Kühlgasstromes in zwei Teilströme vorgesehen ist und dass eine Vielzahl gleichmäßig über den Umfang des Prillturmes (1) angeordneter wabenartiger Gleichrichterelemente vorgesehen (17) sind.

15. Vorrichtung nach Anspruch 14, **dadurch gekennzeichnet, dass** vor dem Eintrag der Bisphenol-A-Schmelze ein Schmelzefilter verwendet wird.

16. Vorrichtung nach Anspruch 15, **dadurch gekennzeichnet, dass** als Schmelzefilter ein Metallgewebesieb mit einer Maschenweite kleiner 80 µm vorgesehen ist.

17. Vorrichtung nach Anspruch 14 bis 16, **dadurch gekennzeichnet, dass** zur Temperierung der Bisphenol-A-Schmelze ein Schmelzekühler (21) vorgesehen ist.

18. Vorrichtung nach Anspruch 17, **dadurch gekennzeichnet, dass** als Schmelzekühler (21) ein Rohrbündelkühler vorgesehen ist.

19. Vorrichtung nach Anspruch 18, **dadurch gekennzeichnet, dass** als Rohrbündelkühler ein 3-Kammer-Rohrbündelaustauscher vorgesehen ist.

20. Vorrichtung nach einem der Ansprüche 14 bis 19, **dadurch gekennzeichnet, dass** die Düsenplatte (2) sphärisch gewölbt ausgebildet ist.

21. Vorrichtung nach einem der Ansprüche 14 bis 20, **dadurch gekennzeichnet, dass** im Kopfbereich des Prillturmes (1) eine Mehrzahl auf einem Lochkreis (19) symmetrisch angeordneter Entnahmerohre (20) zur Kühlgasabfuhr vorgesehen sind.

22. Vorrichtung nach Anspruch 21, **dadurch gekennzeichnet, dass** die mehreren Entnahmerohre (20) außerhalb des Prillturmes (1) symmetrisch zu einem einzigen Entnahmerohr zusammengefaßt sind.

## Claims

1. Process for the preparation of bisphenol-A prills, wherein molten bisphenol-A is charged into a prilling tower at the top of the prilling tower via a nozzle plate with a plurality of nozzles, into which prilling tower a cooling gas which is conducted in a circuit is introduced in counter-flow and wherein the bisphenol-A prill which has cooled to approximately room temperature is collected at the base of the prilling tower and is withdrawn, **characterised in that** the charge of the bisphenol-A melt and the introduction of the cooling gas are effected uniformly in distributed manner over the cross-section of the prilling tower, an annular line which is guided horizontally into the prilling tower (1) and which tapers in cross-section being provided for uniform feed of cooling gas, a baffle plate (14) for dividing the current of cooling gas into two partial currents being provided in the radially flange-mounted inlet of the cooling-gas channel, and a plurality of honeycombed rectifier elements being provided (17), arranged uniformly over the periphery of the prilling tower (1).

2. Process according to Claim 1, **characterised in that** the temperature of the bisphenol-A melt at the input amounts to 160 °C to 170 °C.

3. Process according to Claim 2, **characterised in that** the temperature of the bisphenol-A melt at the input amounts to 165 °C.

4. Process according to any one of Claims 1 to 3, **characterised in that** a purification of the melt is undertaken in a melt filter immediately prior to the charge of the bisphenol-A melt into the prilling tower.

5. Process according to any one of Claims 1 to 4, **characterised in that** the nozzle plate is operated under a slight excess pressure in the range from 0.05 bar to 1 bar.

6. Process according to Claim 5, **characterised in that** the excess pressure amounts to 0.15 bar to 0.3 bar.

7. Process according to any one of Claims 1 to 6, **characterised in that** the cooling gas is conducted over the entire height of the prilling tower in a substantially directed and twist-free flow path.

8. Process according to any one of Claims 1 to 7, **characterised in that** the cooling gas in the upper part of the prilling tower is withdrawn through several discharge pipes which are uniformly distributed over the cross-section of the prilling tower.

9. Process according to Claim 8, **characterised in that** the discharge pipes are subject to attendant heating.

10. Process according to any one of Claims 1 to 9, **characterised in that** the cooling gas which is conducted in a circuit is mechanically filtered.

11. Process according to any one of Claims 1 to 10, **characterised in that** the cooling gas which is conducted in a circuit is continuously cooled.

12. Process according to Claim 11, **characterised in that** cooling of the cooling gas is undertaken in a washing tower with secondary cooling circuit.

13. Process according to Claim 12, **characterised in that** cooling of the cooling gas which is conducted in a circuit is undertaken with demineralised water without dissolved oxygen.

14. Device for the preparation of bisphenol-A prills by implementing the process according to any one of Claims 1 to 13, with a prilling tower, a bisphenol-A melt feed, a cooling-gas circuit with fan and cooler, a bisphenol-A prill drain, **characterised in that** an annular line which is guided horizontally into the prilling tower (1) and which tapers in cross-section is provided for uniform feed of cooling gas, **in that** a baffle plate (14) for dividing the current of cooling gas into two partial currents is provided in the radially flange-mounted inlet of the cooling-gas channel and **in that** a plurality of honeycombed rectifier elements (17) are provided, arranged uniformly over the periphery of the prilling tower (1).

15. Device according to Claim 14, **characterised in that** use is made of a melt filter upstream of the input of the bisphenol-A melt.

16. Device according to Claim 15, **characterised in that** a metallic-cloth sieve with a mesh size less than 80 µm is provided by way of melt filter.

17. Device according to any one of Claims 14 to 16, **characterised in that** a melt cooler (21) is provided for the purpose of temperature control of the bisphenol-A melt.

18. Device according to Claim 17, **characterised in that** a shell-and-tube cooler is provided by way of melt cooler (21).

19. Device according to Claim 18, **characterised in that** a 3-chamber shell-and-tube exchanger is provided by way of shell-and-tube cooler.

20. Device according to any one of Claims 14 to 19, **characterised in that** the nozzle plate (2) is spherically domed.

21. Device according to any one of Claims 14 to 20, **characterised in that** a plurality of discharge pipes (20) arranged symmetrically on a hole circle (19) are provided in the top region of the prilling tower (1) for the drainage of cooling gas.

22. Device according to Claim 21, **characterised in that** the several discharge pipes (20) are combined symmetrically outside the prilling tower (1) into a single discharge pipe.

## Revendications

1. Procédé de fabrication de granules de bisphénol-A, du bisphénol-A liquide de fusion étant introduit en tête d'une colonne de granulation via une plaque porte-filières comportant un grand nombre de filières, un gaz de refroidissement circulant en circuit fermé étant introduit à contre-courant et les granules de bisphénol-A refroidis approximativement à température ambiante étant collectés et extraits au pied de la colonne de granulation, **caractérisé en ce que** l'introduction de la masse fondue de bisphénol-A et l'introduction du gaz de refroidissement se font de manière régulière sur toute la section de la colonne de granulation, une tuyauterie sous forme d'espace annulaire horizontale et de section se rétrécissant étant prévue dans la colonne de granulation
(1) pour la distribution régulière du gaz de refroidissement, une tôle de séparation (14) étant prévue dans l'entrée de canal du gaz de refroidissement fixée radialement à l'aide de brides pour diviser le flux de gaz de refroidissement en deux flux partiels et un grand nombre d'éléments redresseurs en forme de nid d'abeilles (17) étant prévus, répartis régulièrement à la périphérie de la colonne de granulation (1).

2. Procédé suivant la revendication 1, **caractérisé en ce que** la température de la masse fondue de bisphénol-A à l'entrée est de 160°C à 170°C.

3. Procédé suivant la revendication 2,
**caractérisé en ce que** la température de la masse fondue de bisphénol-A à l'entrée est de 165°C.

4. Procédé suivant l'une des revendications 1 à 3, **caractérisé en ce qu'**un nettoyage de la masse fondue a lieu dans un filtre à masse fondue immédiatement avant l'introduction de la masse fondue de bisphénol-A dans la colonne de granulation.

5. Procédé suivant l'une des revendications 1 à 4, **caractérisé en ce que** la plaque porte-filières est exploitée sous une faible surpression de l'ordre de 0,05 à 1 bar.

6. Procédé suivant la revendication 5, **caractérisé en ce que** la surpression est de 0,15 à 0,3 bar.

7. Procédé suivant l'une des revendications 1 à 6, **caractérisé en ce que** le gaz de refroidissement est guidé sur toute la hauteur de la colonne de granulation dans un écoulement essentiellement orienté et exempt de torsion.

8. Procédé suivant l'une des revendications 1 à 7, **caractérisé en ce que** le gaz de refroidissement dans la partie supérieure de la colonne de granulation est extrait à l'aide de plusieurs tuyaux de prélèvement régulièrement répartis sur la section de la colonne de granulation.

9. Procédé suivant la revendication 8, **caractérisé en ce que** les tuyaux de prélèvement sont chauffés.

10. Procédé suivant l'une des revendications 1 à 9, **caractérisé en ce que** le gaz de refroidissement circulant en circuit fermé est filtré mécaniquement.

11. Procédé suivant l'une des revendications 1 à 10, **caractérisé en ce que** le gaz de refroidissement circulant en circuit fermé est refroidi en continu.

12. Procédé suivant la revendication 11, **caractérisé en ce que** le refroidissement du gaz de refroidissement se fait dans une tour de lavage avec circuit secondaire de refroidissement.

13. Procédé suivant la revendication 12, **caractérisé en ce que** le refroidissement du gaz de refroidissement circulant en circuit fermé se fait avec de l'eau déminéralisée sans oxygène dissous.

14. Dispositif de fabrication de granules de bisphénol-A par réalisation du procédé suivant l'une des revendications 1 à 13, avec une colonne de granulation, une introduction de masse fondue de bisphénol-A, un circuit de gaz de refroidissement avec ventilateur et refroidisseur, une extraction de granules de bisphénol-A, **caractérisé en ce qu'**une tuyauterie sous forme d'espace annulaire horizontale et de section se rétrécissant est prévue dans la colonne de granulation (1) pour la distribution régulière du gaz de refroidissement, **en ce qu'**une tôle de séparation (14) est prévue dans l'entrée de canal du gaz de refroidissement fixée radialement à l'aide de brides pour diviser le flux de gaz de refroidissement en deux flux partiels et **en ce qu'**un grand nombre d'éléments redresseurs en forme de nid d'abeilles (17) sont prévus, répartis régulièrement à la périphérie de la colonne de granulation (1).

15. Dispositif suivant la revendication 14, **caractérisé en ce qu'**on utilise un filtre à matière fondue avant l'introduction de la masse fondue de bisphénol-A.

16. Dispositif suivant la revendication 15, **caractérisé en ce qu'**un tamis à tissu métallique d'une ouverture de maille inférieure à 80 µm est prévu comme filtre à matière fondue.

17. Dispositif suivant la revendication 14, **caractérisé en ce qu'**on utilise un refroidisseur à matière fondue (21) pour la températion de la matière fondue de bisphénol-A.

18. Dispositif suivant la revendication 17, **caractérisé en ce que** le refroidisseur à matière fondue (21) est un refroidisseur à faisceau tubulaire.

19. Dispositif suivant la revendication 18, **caractérisé en ce qu'**on utilise comme refroidisseur à faisceau tubulaire un échangeur à faisceau tubulaire à 3 chambres.

20. Dispositif suivant l'une des revendications 14 à 19, **caractérisé en ce que** la plaque porte-filières (2) est construite avec un bombement sphérique.

21. Dispositif suivant l'une des revendications 14 à 20, **caractérisé en ce qu'**une majorité de tuyaux de prélèvement (20) disposés symétriquement sur un cercle de trous (19) sont prévus dans la zone de tête de la colonne de granulation (1) pour l'évacuation du gaz de refroidissement.

22. Dispositif suivant la revendication 21, **caractérisé en ce que** les tuyaux de prélèvement (20) sont regroupés symétriquement en un tuyau de prélèvement unique en dehors de la colonne de granulation (1).
